# EUROPÄISCHE PATENTSCHRIFT

(11) **EP 0 698 379 B1**
(45) Veröffentlichungstag und Bekanntmachung des Hinweises auf die Patenterteilung: **17.04.2002**
(21) Anmeldenummer: 95112825.5
(22) Anmeldetag: 16.08.1995
(51) Int. Cl.: A61F 2/06

(54) **Textile Gefässprothese, Verfahren zu ihrer Herstellung und Werkzeug zu ihrer Herstellung**
Textile vascular prothesis, process and device for producing it
Prothèse vasculaire textile, procédé et dispositif pour sa fabrication

(30) Priorität: 27.08.1994 DE 4430485
(43) Veröffentlichungstag der Anmeldung: 28.02.1996
(73) Patentinhaber: Aesculap AG & Co. KG, 78532 Tuttlingen (DE)
(72) Erfinder: Goldmann, Helmut, Dr., D-34212 Melsungen (DE); Waldert, Helmut, D-34212 Melsungen (DE)
(74) Vertreter: Patentanwälte Ruff, Wilhelm, Beier, Dauster & Partner

(56) Entgegenhaltungen:
- EP-A- 0 391 586
- WO-A-82/01647
- DD-A- 24 242
- DE-A- 2 255 743
- FR-A- 2 334 488

## Beschreibung

Die Erfindung betrifft eine textile, schlauchförmige Gefäßprothese mit verschieden großen Durchmessern an ihren Enden und konischer Ausbildung in Längsrichtung, wobei sie über ihre gesamte Länge eine gleichbleibende Garn- bzw. Fadenzahl im Querschnitt aufweist, ein Verfahren zu ihrer Herstellung sowie eine Kombination aus Prothesenrohling und Lehre zur Durchführung des Verfahrens.

Künstliche Gefäßprothesen zum Ersatz von Blutgefäßen und anderen Hohlorganen des menschlichen und tierischen Körpers sind bekannt. Es gibt gestrickte Gefäßprothesen, vergl. DE-A 24 61 370, es gibt gewirkte Gefäßprothesen, vgl. US-A 39 45 052 und US-A 40 047 252 sowie gewebte Prothesen, vgl. EP-B1 0 108 171. Die Prothesen können eine glatte Oberfläche haben, sie können als Einfachvelour oder als Doppelvelour ausgebildet sein, wie sich aus den obigen Druckschriften ergibt. Ferner können die Prothesen eine sog. Bifurkation besitzen, d.h. eine Aufgabelung in in der Regel zwei dünnere Äste. Die bekannten Prothesen bestehen aus zylindrischen Schlauchabschnitten mit gleichbleibendem Durchmesser, abgesehen von einer Durchmesserverringerung in Folge einer Bifurkation. Die Prothesen können ferner gewellt oder gerippt sein, was in der Fachsprache als Plissierung bezeichnet wird. Die Prothesen können zur Abdichtung der textilen Wandstruktur mit einem resorbierbaren oder nichtresorbierbaren Dichtungsmittel imprägniert sein. Sie können auch in unimprägniertem Zustand eingesetzt werden, wobei während der Operation mindestens eine Vorkoagulierung der Prothesenwandung mit Patientenblut vorgenommen wird, um die erforderliche Abdichtung zu erzielen, bis das Bindegewebe in die Wandstruktur eingewachsen ist.

Um die Strömungsverhältnisse günstig zu gestalten, können Gefäßprothesen vergleichbar mit natürlichen Blutgefäßen konisch ausgebildet sein. Eine solche konische Gefäßprothese aus nichttextilem Material und ihre Herstellung ist in der EP-A2 0 391 586 beschrieben. Zu ihrer Herstellung wird eine extrudierte Schlauchware aus Polytetrafluorethylen zunächst auf das 2 bis 6fache ihrer originalen Länge gestreckt und wird dabei porös. Anschließend erfolgt eine Aufweitung des porösen Schlauchmaterials durch aufweitendes Aufschieben auf einen konischen Dorn, der mit Ultraschall angeregt ist. Eine ähnliche nicht-textile konische Gefäßprothese ist in der DD 160857 beschrieben. Es sind auch textile Gefäßprothesen mit abgestuftem Durchmesser im Handel, ohne daß deren Herstellungsweise bekannt ist. Solche Prothesen sind fälschlicherweise auch als konisch bezeichnet worden. Aus der DE-OS 22 55 743 ist eine textile Gefäßprothese mit verschieden großen Durchmessern an ihren Enden bekannt, die in Längsrichtung konisch ausgebildet ist. Die Gefäßprothese wird nach allgemein bekannten Strick- und Wirktechnologien hergestellt. Insbesondere wird sie auf einer Flachstrickmaschine feiner Teilung in einer der geforderten lichten Grundweite entsprechenden Nadelzahl hergestellt. Dabei werden die Maschen nacheinander abgenommen und in inaktiven Nadeln ausgesetzt, so daß die lichte Weite am Ende der geforderten lichten Weite der Prothese entspricht.

Eine weitere textile Gefäßprothese mit Durchmesservariation ist aus der Druckschrift PLANCK, HEINRICH: Entwicklung einer textilen Arterienprothese mit faserförmiger Struktur. Dissertation Universität Stuttgart, 1980, Seite 38, bekannt. Die Durchmesservariation wird dadurch erzielt, daß mit Hilfe zusätzlicher Bindefäden das freie Lumen der gewirkten Schlauchprothese eingestellt wird. Mit der gleichen Technik können konische Prothesen hergestellt werden.

Aus der WO-A-82/01647 (Seite 12) bekannt ist weiterhin eine geflochtene Gefäßprothese aus Scharen von parallel nebeneinanderliegenden monofilen Fäden. Ein solches Geflecht ermöglicht problemlos Durchmesserveränderungen in Längsrichtung, ohne die Anzahl der monofilen Fäden zu verändern. Da die parallelen monofilen Fäden gegeneinander verschieblich sind, reicht eine Änderung des Flechtwinkels aus, um Durchmesserveränderungen herbeizuführen.

Der Erfindung liegt die Aufgabe zugrunde, eine textile konische Gefäßprothese zu schaffen, die die Variations- und Gestaltungsmöglichkeiten der bisher bekannten textilen Gefäßprothesen bieten soll. Gegenstand der Erfindung ist eine textile, schlauchförmige Gefäßprothese mit verschieden großen Durchmessern an ihren Enden und konischer Ausbildung in Längsrichtung, wobei sie über ihre gesamte Länge eine gleichbleibende Garn- bzw. Fadenzahl im Querschnitt aufweist. Diese Gefäßprothese ist
a) eine gewebte Gefäßprothese und die konische Ausbildung ist durch Schrumpf und durch thermische Fixierung der konischen Form erhältlich oder sie ist
b) eine gewirkte Gefäßprothese und die konische Form ist durch elastische Aufweitung des textilen Gefüges und/oder durch Schrumpf und durch thermische Fixierung der konischen Form erhältlich.
Unter in Längsrichtung konischer Ausbildung wird eine im Betrag stetige Durchmesserveränderung verstanden, die über die Länge der Prothese vorzugsweise kontinuierlich verläuft.

Die erfindungsgemäße Prothese ist gewebt oder gewirkt, je nachdem, welche Anforderungen an sie gestellt werden. Es können die üblichen textilen Materialien Anwendung finden, wobei solche aus Polyester und Polypropylen bevorzugt sind. In der Regel ist die Prothese aus multifilen Garnen gefertigt. Diese können glatt oder texturiert sein. Die Garne können, insbesondere bei gewebten Prothesen, worauf später noch eingegangen wird, mindestens zum Teil hoch schrumpffähig sein.

Gemäß der Erfindung ist es vorgesehen, ausgehend von einem in bekannter Weise als zylindrische Schlauchware hergestellten Prothesenrohling, die konische Form bei der Konfektionierung der Prothese auszubilden. Dadurch ist es bedingt, daß die Garnzahl im Querschnitt der Prothese über die ganze Länge trotz der konischen Ausbildung konstant ist. Dies bedeutet, daß bei gewirkten Prothesen die Stäbchenzahl bzw. die Maschenzahl über die Länge der Prothese konstant ist und bei gewebten Prothesen in entsprechender Weise die Garnzahl im Querschnitt über die gesamte Länge gleichbleibend ist. Die Konizität ist bei gewirkten Prothesen durch elastische Aufweitung und Fixierung und/oder Schrumpf von schrumpffähigem Wandungsmaterial, ausgehend von zylindrischer Schlauchware, ausgebildet. Bei gewebten Prothesen ist die Konizität durch Schrumpf erhältlich.

Die Konizität der Prothese verläuft über ihre gesamte Länge vorzugsweise linear, d.h. sie ist gleichmäßig ausgebildet. Der Durchmesserunterschied von einem Ende zum anderen Ende der Prothese kann 10 bis 100 %, insbesondere 30 bis 50 %, bezogen auf den kleineren Durchmesser, betragen. Die Länge der Prothese kann im Bereich von 10 bis 100 cm liegen. In der Regel handelt es sich um standardisierte Längen von 40 bis 60 cm, insbesondere 40 bzw. 60 cm. Die Längenangabe bezieht sich auf die Länge der Schlauchware ohne eine etwaige Plissierung, die gemäß der Erfindung ebenfalls vorgesehen sein kann und bevorzugt ist. Auch bei plissierten Prothesen verläuft die Konizität entsprechend wie bei nicht plissierten Prothesen, wobei der konische Verlauf durch die gewellte bzw. gefaltete Struktur überlagert ist. Die Plissierung umfaßt in der Regel 15 bis 40, insbesondere 20 bis 30 Wellen bzw. Falten pro 100 mm gestreckte Prothese.

Bei der erfindungsgemäßen konischen Prothese liegt beim Faden- bzw. Garnmaterial im Bereich des Prothesenendes mit großem Durchmesser im Vergleich zum Faden- bzw. Garnmaterial im Bereich des Prothesenendes mit kleinem Durchmesser keine Dehnung oder Streckung des Garn- bzw. Fadenmaterials vor. Dadurch wird erreicht, daß die Zugfestigkeiten der Fasern bzw. Fäden und der Berstdruck der Prothese über ihre Länge im wesentlichen konstant bleibt. Wird der größere Durchmesser durch Aufweitung der gewirkten Schlauchware ausgebildet, dann beschränkt sich diese Aufweitung auf eine Aufweitung bzw. Umorientierung der Garnbindung bzw. Masche, ohne daß das Faden- bzw. Fasermaterial selbst verlängert bzw. gedehnt wird. Durch die Umorientierung der Maschen bzw. Bindungen kann die Porosität der textilen Wandung der Prothese im Bereich des größeren Durchmessers, je nach Prozentsatz der Aufweitung, beginnend etwa in der Prothesenmitte, signifikant größer sein als im Bereich des dünnen Endes, ohne daß sich diese größere Porosität nachteilig auswirkt. Sowohl bei einer Vorkoagulation als auch bei einer Imprägnierung wirken sich die Porositätsunterschiede nicht aus. Entsprechendes gilt bei einer anderen Ausführungsform der Erfindung, bei der der kleinere Durchmesser am dünneren Ende der Prothese durch Schrumpfung von schrumpffähigen Garnen ausgebildet ist, ausgehend von einer Schlauchware, deren Durchmesser dem Durchmesser am größeren Ende entspricht. Bei dieser Ausführungsform kann die Porosität am dünneren Ende, beginnend wiederum etwa im Bereich der Mitte der Prothese, signifikant geringer sein als am Ende mit größerem Durchmesser. Es sind auch Ausführungsformen möglich, bei denen von einer gewirkten Schlauchware ausgegangen wird, die in etwa dem mittleren Durchmesser entspricht und bei der die eine Hälfte durch aufweitende Umorientierung der Bindung aufgeweitet und die andere Hälfte durch zunehmende Schrumpfung verjüngt ist.

Die Abweichung der Durchmesserwerte vom Soll-Wert kann sehr gering gehalten werden und liegt normalerweise maximal bei ±5 %. Die Durchmessergrößen können den üblichen Bereich einschließen, der von 4 mm bis 40 mm reichen kann.

Das Verfahren zur Herstellung der erfindunggemäßen konischen Prothesen ist dadurch gekennzeichnet, daß textile, gewebte oder gewirkte, schlauchförmige Prothesenrohlinge mit über ihre Länge gleichbleibendem Durchmesser im wesentlichen ohne Verlängerung von Fasern bzw. Fäden in eine konische Form überführt und die konische Form durch Erwärmung auf eine Temperatur über dem Glaspunkt und unter dem Schmelzbereich des Fasermaterials und anschließendes Abkühlen fixiert wird, wobei gewebte Prothesenrohlinge durch Schrumpf in die konische Form überführt werden und gewirkte Prothesenrohlinge durch elastische Aufweitung des textilen Gefüges und/oder durch Schrumpf. Die Fixierung wird mit Vorteil durchgeführt, während sich die schlauchförmigen Prothesenrohlinge in vorzugsweise ausgestrecktem Zustand auf einer konischen Lehre befinden. Das Aufbringen der Prothesenrohlinge auf die konische Lehre erfolgt vorzugsweise ohne Einwirkung von Wärme.

Gewirkte Prothesenrohlinge werden bei einer bevorzugten Ausführungsform mit einem Kaliber eingesetzt, dessen Innenumfang dem Umfang der konischen Lehre am kleineren Ende bzw. einer dort vorgesehenen Markierung entspricht, wobei beim Aufbringen der Prothesenrohlinge auf die Lehre eine elastische Aufweitung im textilen Gefüge, insbesondere der Maschen, ohne Faser- bzw. Fadendehnung vorgenommen wird. Die elastische Aufweitung kann dann durch die Wärmeeinwirkung fixiert werden, wobei Rückstellkräfte abgebaut werden. Als konische Lehre kann ein konischer Flachstab verwendet werden, dessen Umfang dem gewünschten Kaliber der fertigen konischen Prothese entspricht. Ist eine Plissierung erwünscht, dann wird diese vorzugsweise vorgebildet, während sich der Prothesenrohling auf dem konischen Flachstab befindet. Hierzu können auf beiden Seiten des konischen Flachstabes in der Schlauchwand in Abständen voneinander vorgesehene querverlaufende Heißprägungen vorgenommen werden, die die späteren Vertiefungen der Plissierung vorbilden. Die querverlaufenden Heißprägungen brauchen nicht den gesamten Umfang des Prothesenschlauches zu erfassen, auch wenn dies die Regel ist. Es reicht eine teilweise Vorbildung der Vertiefungen aus. Gemäß einer weiteren Ausbildung des erfindungsgemäßen Verfahrens ist es vorgesehen, die Größe der Prägestreifen trotz der konischen Ausbildung der auf den konischen Flachstab aufgezogenen Prothese über die gesamte Länge gleichzuhalten. Der Ausdruck Ende des Flachstabes bedeutet nicht, daß der Flachstab hier jeweils aufhört. Es kann in diesem Bereich am konischen Flachstab auch eine Markierung oder Breitenstufe vorgesehen sein, wobei eine Verjüngung der Breite bevorzugt ist, da dies noch das Anbringen von Klemmeinrichtungen begünstigt, die zur Längenfestlegung der Prothese auf dem Flachstab vorgesehen sein können.

Gewebte Prothesenrohlinge werden gemäß einer anderen Ausführungsform des erfindungsgemäßen Verfahrens mit einem Kaliber eingesetzt, dessen Innenumfang dem Umfang der konischen Lehre am größeren Ende entspricht. Die auf die konische Lehre aufgezogenen Prothesenrohlinge können dann durch Ausnutzung eines Faserschrumpfes von schrumpffähigen Fasern unter Kaliberverjüngung auf die konische Lehre aufgeschrumpft werden. Als konische Lehre wird bei dieser Ausführungsform vorzugsweise ein konischer Rundstab verwendet. Ist auch hier eine Plissierung erwünscht, dann kann diese vorzugsweise gleichzeitig mit der Schrumpfung des Kalibers vorgebildet werden. Für diese Vorbildung eignen sich konische Lehren in Form von Rundstäben mit querverlaufenden Wellungen oder Rippen, deren Abstände denjenigen der Plissierung entsprechen.

Die Ausbildung einer Plissierung nach Erzeugung der konischen Form durch Schrumpfen auf einem konischen Flachstab mittels sonst üblicher Verfahren durch Einfaltung der Prothesen ist ebenfalls möglich.

Die Fertigstellung der Plissierung kann vorgenommen werden, indem die konisch geformte Prothese mit der vorgebildeten Plissierung unter Ausbildung eines Faltenbalges auf einen konischen Rundstab aufgeschoben wird, dessen Konizität entsprechend der durch den Faltenbalg bedingten Verkürzung steiler ist. Es kann dann eine erneute Fixierung durch Erwärmung zum Abbau von Rückstellkräften vorgenommen werden. Nach Erkalten wird dann die fertige Prothese mit bleibender Plissierung abgenommen. Ihre Länge ist entsprechend kleiner als die der nicht plissierten Prothese. Ist die Plissierung der Prothese auf einem Flachstab durch Prägung vorgebildet worden, dann ist dies in der Regel an der fertigen, nicht oder noch nicht imprägnierten, Prothese dadurch erkennbar, daß die Prothese beim Strecken unter starkem Zug dazu neigt, eine Flachform anzunehmen, die der Lage der Prothese auf dem Flachstab entspricht.

Konische Prothesen mit Bifurkation können in entsprechender Weise hergestellt werden. Hierzu können für den Hauptschlauch mit größerem Durchmesser und für die abgezweigten Schläuche mit kleinerem Durchmesser getrennte Lehren und/oder eine entsprechend gegabelte Lehre verwendet werden.

Die Erfindung betrifft auch eine Kombination aus Prothesenrohling und Lehre zur Herstellung der konischen Prothese, die dadurch gekennzeichnet ist, daß eine konische Lehre vorgesehen ist, auf die ein vorgebildeter textiler Prothesenrohling, der einen über seine Länge gleichbleibenden Durchmesser hat, ohne Verlängerung des Faden- bzw. Fasermaterials aufschiebbar ist, wobei der ursprüngliche Innenumfang des Prothesenrohlings und der Umfang der konischen Lehre am größeren bzw. kleineren Ende so aufeinander abgestimmt sind, daß der Innenumfang des Prothesenrohlings dem Umfang der konischen Lehre im Bereich zwischen großem Ende und kleinem Ende entspricht. Bei einer Ausführungsform wird die Lehre von einem konischen Flachstab gebildet, dessen Umfang am schmalen Ende vorzugsweise dem Innenumfang des Prothesenrohlings entspricht. Bei einer anderen Ausführungsform wird die Lehre von einem vorzugsweise gewellten bzw. gerippten konischen Rundstab gebildet, dessen Umfang am größeren Ende vorzugsweise dem Innenumfang des Prothesenrohlings entspricht.

Weitere Merkmale und Vorteile der Erfindung ergeben sich aus der nachfolgenden Beschreibung von bevorzugten Ausführungsformen in Verbindung mit der Zeichnung und den Unteransprüchen. Hierbei können die einzelnen Merkmale bei den einzelnen Ausführungsformen jeweils für sich allein oder in Kombination miteinander verwirklicht sein. In der Zeichnung zeigen:
- Fig. 1: eine Ausführungsform der erfindungsgemäßen konischen Prothese,
- Fig. 2: eine Vorrichtung zur Herstellung der Prothese mit halb aufgeschobenem Prothesenrohling, und
- Fig. 3: eine andere Vorrichtung zur Herstellung der Prothese mit vollständig aufgeschobenem Prothesenrohling.

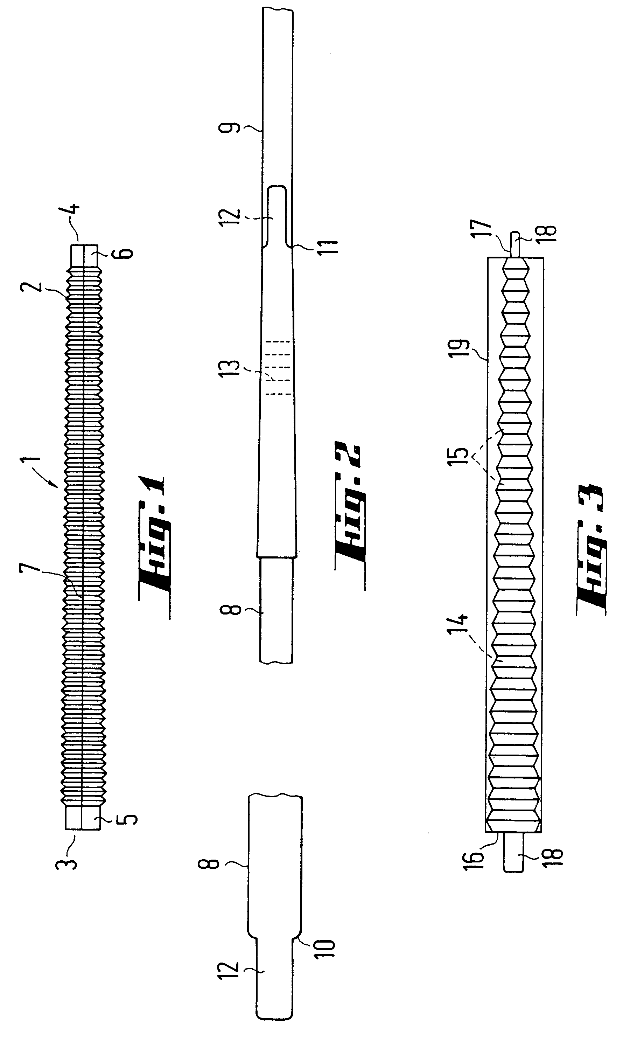

Die in Fig. 1 dargestellte Ausführungsform der Erfindung zeigt eine textile konische Gefäßprothese 1, die aus Polyestergarn als Doppelvelour-Schlauchware gewirkt ist. Die Schlauchware hatte ursprünglich eine Länge von 60 cm und ist durch wellenförmige Plissierung 2 des Wandungsmaterials auf etwa die Hälfte verkürzt. An einem Ende 3 besitzt die Prothese einen größeren Durchmesser entsprechend einem Kaliber von 8 mm, am anderen Ende 4 kleineren Durchmessers beträgt das Kaliber 6 mm. Die Konizität verläuft gleichmäßig linear von einem Ende zum anderen, wobei die Durchmesserabweichungen im Bereich von 0,5 % liegen. Die Maschenzahl bzw. Stäbchenzahl entlang des Prothesenumfangs ist von einem Ende bis zum anderen Ende gleichbleibend, wobei die Maschengröße vom kleineren Ende 4 zum größeren Ende 3 etwas zunimmt. Die Endabschnitte 5 und 6 an den Enden 3 und 4 sind im wesentlichen zylindrisch ausgebildet und weisen keine Plissierung auf. Diese Endabschnitte dienen bei der Herstellung der Prothese zur mechanischen Festlegung. Sie werden zum Beispiel nach einer möglichen Imprägnierung mit einer resorbierbaren oder nichtresorbierbaren Beschichtung und/oder der Konfektionierung vor dem Sterilisieren abgeschnitten. Eine Kennlinie 7 ist in Form von schwarzem Garnmaterial der im übrigen weißen Prothese eingewirkt. Sie dient dazu, die Ausrichtung der Prothese auch bei der Operation erkennen zu können, um eine Verdrehung und dadurch möglicherweise bedingte Durchmesserverkleinerung der Prothese bei der Implantation zu vermeiden.

Fig. 2 zeigt eine konische Lehre 8 für die konische Aufweitung eines Prothesenrohlings 9, der als Schlauchware mit konstantem Durchmesser gewirkt ist. Die konische Lehre hat die Form eines konisch verjüngten Flachstabes 8 mit einem breiten Ende 10 und einem schmalen Ende 11, wobei an den Enden jeweils abgesetzte Verlängerungsstücke 12 vorgesehen sind. Die Schlauchware des Prothesenrohlings 9 hat ein Kaliber von 6 mm entsprechend einem Innenumfang von ca. 19 mm. Dementsprechend ist die Breite und Dicke des Flachstabes 8 am schmalen Ende 11 so gehalten, daß sich in etwa ein entsprechender Außenumfang ergibt. Die Lehre dient zur Aufweitung des Prothesenrohlinges auf ein Kaliber 8 mm am breiten einen Ende. Dementsprechend sind Breite und Dicke des Flachstabes am breiten Ende so gehalten, daß sich ein Umfang von ca. 25 mm ergibt. Das Aufschieben des Prothesenrohlings 9 auf die konische Lehre 8 erfolgt bei Raumtemperatur unter elastischer Aufweitung der Maschenstruktur des Gewirkes. Der aufgeschobene Prothesenrohling 9 ist etwas länger als der konische Abschnitt der Lehre 8, so daß seine Enden an den Verlängerungsstücken 12 befestigt werden können. In dieser Lage wird die konische Form des Prothesenrohlings fixiert, um Rückstellkräfte abzubauen. Die Fixiertemperaturen können bei Polyester im Bereich von 80 bis 200 °C, insbesondere 150 bis 180 °C liegen, wobei die Fixierzeiten bei 24 Stunden bis 1 Minute, insbesondere 7 bis 3 Minuten liegen. Bei anderen Materialien werden die Fixierbedingungen entsprechend ausgewählt, wobei die Temperatur über dem Glaspunkt und unter dem Schmelzbereich des jeweiligen Polymers liegt. Nach der Fixierung der konischen Form kann eine Vorbildung der Plissierung durch Prägung vorgenommen werden, während sich der Prothesenrohling noch auf der konischen Lehre befindet. Hierzu werden auf beiden Seiten der Lehre parallele Querstreifen 13 auf die Wandung des Prothesenrohlings geprägt. Die Prägestreifen 13 sind in Fig. 2 nur gestrichelt angedeutet, da sie erst nach der konischen Aufweitung des Prothesenrohlings 9 angebracht werden. Die Prägung kann bei Temperaturen im Bereich von 120 bis 200 °C, insbesondere 150 bis 180 °C während einer Zeit von 5 Minuten bis 30 Sekunden, insbesondere 2 bis 1 Minute durchgeführt werden. Der Prägedruck kann in weiten Grenzen variieren und nimmt mit größer werdendem Kaliber des Prothesenrohlings vorzugsweise ab. So kann der Prägedruck bei einem Prothesenrohling mit einem Kaliber von 6 mm im Bereich von 35 N/cm² und bei einem Kaliber von 30 mm im Bereich von 7 N/cm² liegen. Die Länge der geprägten Querstreifen 13 braucht nicht den gesamten Umfang der Prothese zu erfassen. Aus praktischen Gründen entspricht die Länge der Querstreifen 13 in der Regel der Breite des Flachstabes 8.

Fig. 3 zeigt eine andere Ausführungsform einer konischen Lehre. Dort ist eine Lehre in Form eines konischen Rundstabes 14 vorgesehen, der an seiner Oberfläche eine leichte Wellung bzw. Querrippen 15 zur Vorbildung der Plissierung aufweist. Die Lehre hat ein dickes Ende 16 mit Kaliber 8 und ein dünnes Ende 17 mit Kaliber 6, wobei an den Enden wiederum Verlängerungsstücke 18 vorgesehen sind.

Im Gegensatz zur Ausführungsform nach Fig. 2 ist für die Lehre 14 in Form des Rundstabes ein Prothesenrohling vorgesehen, dessen Kaliber dem Kaliber 8 des dicken Endes 16 der Lehre 14 entspricht. Der Prothesenrohling 19 enthält einen Anteil an Fasermaterial mit hoher Schrumpffähigkeit, so daß der Prothesenrohling beim Erwärmen und gleichzeitigen Fixieren auf den konischen Rundstab 14 aufschrumpft und dabei infolge der Querrippen 15 des Rundstabes eine leichte Wellung annimmt, durch die die Plissierung vorgebildet wird.

Wie aus Fig. 3 ersichtlich, ist es auch möglich, einen gewirkten Prothesenrohling zu verwenden, dessen Kaliber etwa im Mittelbereich zwischen dem Kaliber am dünnen und am dicken Ende liegt, so daß der Prothesenrohling teilweise durch Aufweitung, teilweise durch Schrumpf in die konische Form gebracht wird. Die Ausführungsform nach Fig. 3 eignet sich insbesondere auch für gewebte Prothesen, die nur eine geringe elastische Dehnbarkeit der Bindung besitzen, ohne daß die Faser gedehnt wird. Solche gewebte Prothesenrohlinge werden deshalb mit einem Kaliber eingesetzt, der dem dicken Ende der konischen Lehre entspricht, so daß die konische Form im wesentlichen ausschließlich durch Schrumpf erzielt wird.

Nach dem Abnehmen der Prothesenrohlinge von der konischen Lehre können sie auf konische Rundstäbe aufgeschoben werden, deren Länge gegenüber der Länge der ursprünglichen Schlauchware auf etwa ein Viertel verkürzt ist, und die dementsprechend eine stärkere Konizität aufweisen. Zur praktischen Handhabung sind die konischen Rundstäbe vorzugsweise länger gehalten, wobei die gewünschten Kaliber für die beiden Enden der Prothesen durch entsprechende Markierungen auf dem Rundstab festgehalten sind. Beim Aufschieben des konisch geformten Prothesenrohlings mit vorgebildeter Plissierung lassen sich die Prothesenrohlinge zieharmonikaartig unter Ausbildung einers Faltenbalges zusammenschieben. In dieser Lage werden sie nochmals einer Fixierung unterworfen, wobei die Fixiertemperaturen bei Polyester in der Regel zwischen 140 und 180 °C, insbesondere zwischen 150 und 170 °C liegen. Die Fixierdauer kann dementsprechend zwischen 2 Stunden und 10 Minuten, insbesondere 20 bis 15 Minuten liegen. Nach dem Abnehmen behält die Prothese die konische Form und die Plissierung.

Die konischen Prothesen nach der Erfindung können, falls erwünscht, zur Abdichtung der Maschenstruktur in an sich bekannter Weise imprägniert werden. Eine Imprägnierung mit Gelatine, die insbesondere mit Diisocyanat vernetzt wird, ist bevorzugt.

Durchlässigkeits- und Festigkeitsmessungen an aufgeweiteten Prothesen haben gezeigt, daß die Berstfestigkeit der Prothesen durch die Aufweitung keine Veränderung erfährt. Die Wasserdurchlässigkeit der unimpägnierten Prothesen nimmt durch die Aufweitung etwas zu, wobei die Durchlässigkeit vom Ende mit kleinem Kaliber bis zur Mitte der Prothese signifikant zunimmt und von der Prothesenmitte bis zum aufgeweiteten Ende nur noch unwesentlich. Dies ist möglicherweise darauf zurückzuführen, daß die Maschenstruktur in der Prothesenwandung bei der Aufweitung flacher wird, ohne daß die Poren in der Maschenstruktur wesentlich größer werden.

## Patentansprüche

1. Textile, schlauchförmige Gefäßprothese (1) mit verschieden großen Durchmessern an ihren Enden und konischer Ausbildung in Längsrichtung, wobei sie über ihre gesamte Länge eine gleichbleibende Garn- bzw. Fadenzahl im Querschnitt aufweist, **dadurch gekennzeichnet, daß** die Gefäßprothese (1)
a) eine gewebte Gefäßprothese ist und die konische Ausbildung durch Schrumpf und durch thermische Fixierung der konischen Form erhältlich ist oder
b) eine gewirkte Gefäßprothese ist und die konische Form durch elastische Aufweitung des textilen Gefüges und/oder durch Schrumpf und durch thermische Fixierung der konischen Form erhältlich ist.

2. Prothese nach Anspruch 1, **dadurch gekennzeichnet, daß** die Konizität über die gesamte Länge der Prothese im wesentlichen linear ausgebildet ist.

3. Prothese nach Anspruch 1 oder 2, **dadurch gekennzeichnet, daß** der Durchmesserunterschied über eine Prothesenlänge im Bereich von 10 bis 100 cm, bevorzugt von 40 bis 60 cm, 10 bis 100 %, insbesondere 30 bis 50 %, bezogen auf den kleineren Durchmesser, beträgt.

4. Prothese nach einem der vorhergehenden Ansprüche, **dadurch gekennzeichnet, daß** die Durchmesserabweichung vom Soll-Wert maximal ±5 % beträgt.

5. Prothese nach einem der vorhergehenden Ansprüche, **dadurch gekennzeichnet, daß** der Berstdruck der Prothese über die gesamte Länge im wesentlichen konstant ist.

6. Prothese nach einem der vorhergehenden Ansprüche, **dadurch gekennzeichnet, daß** sie eine konisch verlaufende Plissierung aufweist.

7. Verfahren zur Herstellung der Prothese nach einem der vorhergehenden Ansprüche, **dadurch gekennzeichnet, daß** textile, gewebte oder gewirkte, schlauchförmige Prothesenrohlinge mit über ihre Länge gleichbleibendem Durchmesser im wesentlichen ohne Verlängerung von Fasern bzw. Fäden in eine konische Form überführt und die konische Form durch Erwärmung auf eine Temperatur über dem Glaspunkt und unter dem Schmelzbereich des Fasermaterials und anschließendes Abkühlen fixiert wird, wobei gewebte Prothesenrohlinge durch Schrumpf in die konische Form überführt werden und gewirkte Prothesenrohlinge durch elastische Aufweitung des textilen Gefüges und/oder durch Schrumpf.

8. Verfahren nach Anspruch 7, **dadurch gekennzeichnet, daß** die Fixierung durchgeführt wird, während sich die schlauchförmigen Prothesenrohlinge in vorzugsweise ausgestrecktem Zustand auf einer konischen Lehre befinden, und daß vorzugsweise die Prothesenrohlinge ohne Wärmeeinwirkung auf die konische Lehre aufgebracht werden.

9. Verfahren nach Anspruch 8, **dadurch gekennzeichnet, daß** insbesondere gewirkte Prothesenrohlinge mit einem Kaliber eingesetzt werden, deren Innenumfang im wesentlichen dem Umfang am kleineren Ende der konischen Lehre entspricht, und daß beim Aufbringen der Prothesenrohlinge auf die Lehre eine elastische Aufweitung im textilen Gefüge, insbesondere der Maschen, ohne Faserdehnung vorgenommen wird und die Aufweitung dann durch Wärmeeinwirkung fixiert wird.

10. Verfahren nach Anspruch 8 oder 9, **dadurch gekennzeichnet, daß** als Lehre ein Flachstab verwendet wird, dessen Umfang dem gewünschten Kaliber der konischen Prothese entspricht.

11. Verfahren nach Anspruch 10, **dadurch gekennzeichnet, daß** nach dem Fixieren vorzugsweise auf beiden Seiten des konischen Flachstabes eine Plissierung vorbereitet wird, indem Vertiefungen der Plissierung durch Heißprägung von querverlaufenden Streifen vorgebildet werden.

12. Verfahren nach Anspruch 11, **dadurch gekennzeichnet, daß** die geprägten Querstreifen über die gesamte Länge der konischen Prothese die gleiche Größe aufweisen.

13. Verfahren nach Anspruch 8, **dadurch gekennzeichnet, daß** insbesondere gewebte Prothesenrohlinge mit einem Kaliber eingesetzt werden, dessen Innenumfang dem Umfang der konischen Lehre am größeren Ende entspricht und daß die Prothesenrohlinge durch Ausnutzung eines Faserschrumpfes von schrumpffähigen Fasern bzw. Fäden unter Kaliberverjüngung auf die konische Lehre aufgeschrumpft werden.

14. Verfahren nach Anspruch 13, **dadurch gekennzeichnet, daß** als konische Lehre ein konischer Rundstab verwendet wird und daß vorzugsweise gleichzeitig mit der Schrumpfung auch eine Plissierung zumindest vorgebildet wird und hierzu der konische Rundstab querverlaufende Wellungen oder Rippen aufweist.

15. Verfahren nach einem der Ansprüche 7 bis 14, **dadurch gekennzeichnet, daß** die konisch geformte Prothese mit vorgebildeter Plissierung unter Ausbildung eines Faltenbalges auf einen konischen Rundstab aufgeschoben wird, dessen Konizität entsprechend der durch den Faltenbalg bedingten Verkürzung steiler ist und die Prothese dann erneut fixiert wird.

16. Kombination aus Prothesenrohling und Lehre (8; 14) zur Durchführung des Verfahrens nach einem der Ansprüche 7 bis 15, **dadurch gekennzeichnet, daß** eine konische Lehre (8; 14) vorgesehen ist, auf die ein vorgebildeter textiler, einen über seine Länge gleichbleibenden Durchmesser besitzender, Prothesenrohling ohne Verlängerung des Faden- bzw. Fasermaterials aufschiebbar ist, wobei der ursprüngliche Innenumfang des Prothesenrohlings und der Umfang der konischen Lehre (8; 14) am größeren bzw. kleineren Ende (10, 11; 16, 17) so aufeinander abgestimmt sind, daß der Innenumfang des Prothesenrohlings dem Umfang der konischen Lehre (8; 14) im Bereich zwischen großem Ende (10; 16) und kleinem Ende (11; 17) entspricht.

17. Kombination nach Anspruch 16, **dadurch gekennzeichnet, daß** die Lehre (8) von einem konischen Flachstab gebildet wird, dessen Umfang am schmalen Ende (11) vorzugsweise dem Innenumfang des Prothesenrohlings entspricht.

18. Kombination nach Anspruch 16, **dadurch gekennzeichnet, daß** die Lehre von einem vorzugsweise gewellten bzw. gerippten konischen Rundstab (14) gebildet wird, dessen Umfang am größeren Ende (16) vorzugsweise dem Innenumfang des Prothesenrohlings entspricht.

## Claims

1. Textile, tubular vascular prosthesis (1) with different diameters at their ends and a conical structure in the longitudinal direction, whilst having a constant yarn or thread count in cross-section over the entire length thereof, **characterized in that** the vascular prosthesis (1)
a) is a woven vascular prosthesis and the conical formation is obtainable by shrinkage and thermal fixing of the conical shape, or
b) is a warp-knitted vascular prosthesis and the conical shape is obtainable by elastic expansion of the textile structure and/or by shrinkage and thermal fixing of the conical shape.

2. Prosthesis according to claim 1, **characterized in that** the conicity is substantially linear over the entire prosthesis length.

3. Prosthesis according to claim 1 or 2, **characterized in that** the diameter difference over a prosthesis length in the range 10 to 200 cm, preferably 40 to 60 cm, is 10 to 100%, particularly 30 to 50%, based on the smaller diameter.

4. Prosthesis according to one of the preceding claims, **characterized in that** the diameter divergence from the desired value is max. ± 5%.

5. Prosthesis according to one of the preceding claims, **characterized in that** the prosthesis bursting pressure is substantially constant over the entire length.

6. Prosthesis according to one of the preceding claims, **characterized in that** it has a conically directed pleating.

7. Method for the manufacture of the prosthesis according to one of the preceding claims, **characterized in that** textile, woven or warp-knitted, tubular prosthesis blanks with a constant diameter over the length thereof and substantially without lengthening the fibres or threads are transformed into a conical shape and the conical shape is fixed by heating to a temperature above the glass transition point and under the melting range of the fibrous material and subsequent cooling, woven prosthesis blanks being transformed into the conical shape by shrinkage and warp-knitted prosthesis blanks by elastic expansion of the textile structure and/or by shrinkage.

8. Method according to claim 7, **characterized in that** fixing takes place whilst the tubular prosthesis blanks are located in a preferably stretched out state on a conical template and that preferably the prosthesis blanks are applied to the conical template without heat action.

9. Method according to claim 8, **characterized in that** in particular warp-knitted prosthesis blanks are used with a gauge, whose inner circumference substantially corresponds to the circumference at the smaller end of the conical template and that on applying the prosthesis blanks to the template an elastic expansion takes place in the textile structure, particularly the meshes, without thread extension and the expansion is then fixed by heat action.

10. Method according to claim 8 or 9, **characterized in that** the template is constituted by a flat bar, whose circumference corresponds to the desired gauge of the conical prosthesis.

11. Method according to claim 10, **characterized in that** after fixing, a pleating is prepared preferably on both sides of the conical flat bar, **in that** depressions of the pleating are preshaped by hot embossing transversely directed strips.

12. Method according to claim 11, **characterized in that** the embossed transverse strips have the same size over the entire length of the conical prosthesis.

13. Method according to claim 8, **characterized in that** in particular woven prosthesis blanks are used with a gauge, whose inner circumference corresponds to the circumference of the conical template at the larger end and that the prosthesis blanks are shrunk onto the conical template utilizing a fibre shrinkage of shrinkable fibres or threads and accompanied by gauge reduction.

14. Method according to claim 13, **characterized in that** the conical template is constituted by a conical round bar and that preferably simultaneously with the shrinkage a pleating is at least preshaped and for this purpose the conical round bar has transversely directed corrugations or ribs.

15. Method according to one of the claims 7 to 14, **characterized in that** the conically shaped prosthesis with preshaped pleating is engaged on a conical round bar, accompanied by the formation of a bellows and the conicity thereof is steeper corresponding to the shortening resulting from the bellows and the prosthesis is then again fixed.

16. Combination of prosthesis blank and template (8; 14) for performing the method according to one of the claims 7 to 15, **characterized in that** a conical template (8; 14) is provided and onto it can be engaged a preshaped textile prosthesis blank, having a constant diameter over its length and without lengthening the thread or fibrous material, the original inner circumference of the prosthesis blank and the circumference of the conical template (8, 14) at the larger or smaller end (10, 11; 16, 17) being so matched to one another that the inner circumference of the prosthesis blank corresponds to the circumference of the conical template (8; 14) in the area between the large end (10; 16) and small end (11; 17).

17. Combination according to claim 16, **characterized in that** the template (8) is formed by a conical flat bar, whose circumference at the narrow end (11) preferably corresponds to the inner circumference of the prosthesis blank.

18. Combination according to claim 16, **characterized in that** the template is formed by a preferably corrugated or ribbed, conical round bar (14), whose circumference at the larger end (16) preferably corresponds to the inner circumference of the prosthesis blank.

## Revendications

1. Prothèse vasculaire textile tubulaire (1) avec des diamètres de différentes dimensions aux extrémités et une forme conique dans le sens longitudinal, la prothèse présentant en section transversale sur toute la longueur un nombre constant de fils ou filaments, **caractérisée en ce que**
la prothèse vasculaire (1)
a) est une prothèse vasculaire tissée et la forme conique est obtenue par rétraction et par fixation thermique de la forme conique ou
b) est une prothèse vasculaire tricotée et la forme conique est obtenue par élargissement élastique de la structure textile et/ou par rétraction et par fixation thermique de la forme conique.

2. Prothèse selon la revendication 1, **caractérisée en ce que** la conicité est conçue sensiblement linéaire sur toute la longueur de la prothèse.

3. Prothèse selon la revendication 1 ou 2, **caractérisée en ce que** la différence de diamètre sur une longueur de prothèse est comprise entre 10 et 100 cm, de préférence 40 à 60 cm de 10 à 100 %, en particulier de 30 à 50 % par rapport au plus petit diamètre.

4. Prothèse selon l'une des revendications précédentes, **caractérisé en ce que** la différence de diamètre par rapport à la valeur de consigne s'élève au maximum à ± 5 %.

5. Prothèse selon l'une des revendications précédentes, **caractérisé en ce que** la pression d'éclatement de la prothèse est sensiblement constante sur toute la longueur.

6. Prothèse selon l'une des revendications précédentes, **caractérisée en ce qu'**elle présente un plissé s'étendant coniquement.

7. Procédé pour la fabrication de la prothèse selon l'une des revendications précédentes, **caractérisée en ce que** des préformes de prothèse textile, tricotées ou tissées tubulaires avec un diamètre constant sur leur longueur sont transformées en une forme conique sensiblement sans prolongement de fibres respectivement de fils et la forme conique est fixée par réchauffement à une température supérieure au point de vitrification et inférieure à la zone de fusion de la matière fibreuse et par refroidissement consécutif, des préformes de prothèse tissées étant transformées par rétraction en forme conique et des préformes de prothèse tricotée par élargissement élastique de la structure textile et/ou par rétraction.

8. Procédé selon la revendication 7, **caractérisé en ce que** la fixation est exécutée pendant que les préformes de prothèse tubulaire se trouvent de préférence à l'état étiré sur un gabarit conique et que de préférence les préformes de prothèse sont appliquées sur le gabarit conique sans action thermique.

9. Procédé selon la revendication 8, **caractérisé en ce qu'**en particulier des préformes de prothèse tricotées sont utilisées avec un calibre dont la périphérie interne correspond sensiblement à la périphérie de la plus petite extrémité du gabarit conique et **en ce qu'**à l'application des préformes de prothèse sur le gabarit, un élargissement élastique se produit dans la structure textile, en particulier dans les mailles sans dilatation de la fibre et **en ce que** l'élargissement est fixé ensuite par action thermique.

10. Procédé selon la revendication 8 ou 9, **caractérisé en ce qu'**en tant que gabarit, on utilise une baguette plate, dont la périphérie correspond au calibre souhaité de la prothèse conique.

11. Procédé selon la revendication 10, **caractérisée en ce qu'**après la fixation de préférence des deux côtés de la baguette plate conique, un plissé est préparé dans le fait que des profondeurs du plissé sont préformées par estampage à chaud de bandes transversales.

12. Procédé selon la revendication 11, **caractérisée en ce que** les bandes transversales estampées présentent la même dimension sur toute la longueur de la prothèse conique.

13. Procédé selon la revendication 8, **caractérisé en ce qu'**en particulier des préformes de prothèse tissées sont utilisées avec un calibre dont la périphérie interne correspond à la périphérie du gabarit conique à l'extrémité la plus grande et **en ce que** les préformes de prothèse sont rétractées sur le gabarit conique en utilisant la rétraction de fibres respectivement de fils rétractables par rétrécissement du calibre.

14. Procédé selon la revendication 13, **caractérisé en ce qu'**en tant que gabarit conique est utilisée une baguette ronde conique et **en ce que** de préférence au moins un plissé se produit également en même temps que la rétraction et de ce fait, la baguette ronde conique présente des ondulations ou nervures transversales.

15. Procédé selon l'une des revendications 7 à 14, **caractérisé en ce que** la prothèse de forme conique avec un plissé préformé est enfilée sur une baguette ronde conique en formant un soufflet dont la conicité est plus raide par rapport au rétrécissement causé par le soufflet et la prothèse est alors fixée à nouveau.

16. Combinaison de préforme de prothèse et de gabarit (8; 14) pour la mise en oeuvre du procédé selon l'une des revendications 7 à 15, **caractérisée en ce qu'**il est prévu un gabarit conique (8; 14) sur lequel est enfilée une préforme préformée textile de prothèse possédant un diamètre constant sur sa longueur sans prolongement de la matière fibreuse, la périphérie interne initiale de la préforme de prothèse et la périphérie du gabarit conique (8; 14) sur l'extrémité la plus grande respectivement la plus petite (10; 11; 16, 17) étant coordonnées l'une à l'autre de sorte que la périphérie interne de la préforme de prothèse correspond à la périphérie du gabarit conique (8; 14) dans la zone entre la grande extrémité (10; 16) et la petite extrémité (11; 17).

17. Combinaison selon la revendication 16, **caractérisé en ce que** le gabarit (8) est formé par une baguette conique plate dont la périphérie à l'extrémité étroite (11) correspond de préférence à la périphérie interne de la préforme de prothèse.

18. Combinaison selon la revendication 16, **caractérisé en ce que** le gabarit est formé par une baguette ronde conique (14) de préférence nervurée ou ondulée dont la périphérie à la plus grande extrémité (16) correspond de préférence à la périphérie interne de la préforme de prothèse.
